(19) **Europäisches Patentamt**
**European Patent Office**
**Office européen des brevets**

(11) **EP 2 416 153 B1**

(12) **EUROPEAN PATENT SPECIFICATION**

(45) Date of publication and mention of the grant of the patent:
**16.04.2014 Bulletin 2014/16**

(51) Int Cl.:
**G01N 33/28** (2006.01)

(21) Application number: **11176291.0**

(22) Date of filing: **02.08.2011**

(54) **Method for predicting the quality and yields of a crude oil**

Verfahren zur Vorhersage der Rohölqualität und -ausbeute

Procédé pour prédire la qualité et le rendement de pétrole brut

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.08.2010 IT MI20101456**

(43) Date of publication of application:
**08.02.2012 Bulletin 2012/06**

(73) Proprietor: **ENI S.p.A.**
**00144 Rome (IT)**

(72) Inventor: **Pavoni, Silvia**
**Pioltello (MI) (IT)**

(74) Representative: **Maiwald Patentanwalts GmbH**
**Elisenhof**
**Elisenstrasse 3**
**80335 München (DE)**

(56) References cited:
- **BLANCO M ET AL: "Geographical origin classification of petroleum crudes from near-infrared spectra of bitumens", APPLIED SPECTROSCOPY JULY 2001 SOCIETY FOR APPLIED SPECTROSCOPY US, vol. 55, no. 7, July 2001 (2001-07), pages 834-839, XP8132252, DOI: DOI:10.1366/0003702011952857**
- **BLANCO M ET AL: "Determination of physical properties of bitumens by use of near-infrared spectroscopy with neural networks. Joint modelling of linear and non-linear parameters", ANALYST 2001 GB LNKD- DOI: 10.1039/B009255J, vol. 126, no. 3, 2001, pages 378-382, XP7916880, ISSN: 0003-2654**
- **BORGES C ET AL: "Geographical classification of weathered crude oil samples with unsupervised self-organizing maps and a consensus criterion", CHEMOMETRICS AND INTELLIGENT LABORATORY SYSTEMS, ELSEVIER SCIENCE PUBLISHERS B.V. AMSTERDAM, NL, vol. 101, no. 1, 15 March 2010 (2010-03-15), pages 43-55, XP026917022, ISSN: 0169-7439, DOI: DOI:10.1016/J.CHEMOLAB. 2010.01.001 [retrieved on 2010-01-18]**
- **LIAU L C-K ET AL: "Expert system of a crude oil distillation unit for process optimization using neural networks", EXPERT SYSTEMS WITH APPLICATIONS FEBRUARY 2004 ELSEVIER LTD GB, vol. 26, no. 2, February 2004 (2004-02), pages 247-255, XP7916865, DOI: DOI: 10.1016/S0957-4174(03)00139-8**
- **MOTLAGHI S ET AL: "An expert system design for a crude oil distillation column with the neural networks model and the process optimization using genetic algorithm framework", EXPERT SYSTEMS WITH APPLICATIONS, OXFORD, GB, vol. 35, no. 4, 1 November 2008 (2008-11-01), pages 1540-1545, XP023781438, ISSN: 0957-4174, DOI: 10.1016/J.ESWA.2007.08.105 [retrieved on 2008-08-08]**
- **WANG S ET AL: "Predicting saturates of sour vacuum gas oil using artificial neural networks and genetic algorithms", EXPERT SYSTEMS WITH APPLICATIONS, OXFORD, GB, vol. 37, no. 7, 1 July 2010 (2010-07-01), pages 4768-4771, XP026989019, ISSN: 0957-4174 [retrieved on 2009-11-26]**

EP 2 416 153 B1

- 'Combining Genetic Algorithms and Neural Networks: The Encoding Problem', [Online] 01 December 1994, page 1, XP055083697 Retrieved from the Internet: <URL:http://homepages.inf.ed.ac.uk/pkoehn/p ublications/gann94.pdf> [retrieved on 2013-10-11]

**Description**

The present invention relates to the field of the study and analysis of materials for determining their physical and chemical properties and in particular a method for predicting the quality and yields of crude oils.

The possibility of predicting the quality and yields of a crude oil from small quantities of sample is a useful instrument of commercial and operative analysis.

The analyses necessary for characterizing a crude oil in terms of yields and quality of the products obtainable from its primary distillation (GPL, naphtha, gas oil, vacuum distillate, distillation residue), require considerable commitment in terms of both time and equipment.

The possibility of predicting yields and/or physico-chemical characteristics of crude oils, through algorithms which use easily determinable parameters or routine analyses as input data, offers numerous advantages.

The optimum selection of crude oils is an important factor for refining companies, as the quality of the oil is an integrant part of the purchasing plan of the crude oil, and refining companies must find an adequate approach for its management.

Frequent variations in the quality of refinery feedstocks require particular attention to this aspect.

In the oil industry, a reliable prediction system of the yields and quality of crude oil, based on limited and rapidly obtainable analytical results, can be a useful instrument for many purposes:

-    for a rapid overview of the quality of the oils coming from the wells;
-    for mapping the variations in the quality in a reservoir;
-    for predicting the quality of a charge of crude oil;
-    for optimizing refining operations, in particular with crude oils having an extremely variable quality range;
-    for having a rapid idea about the evolutionary tendency of the quality of the crude oil.

Prediction methods of the characteristics of crude oils with standard laboratory analyses are known in the state of the art.

Patent US 5,699,269, for example, describes a method for predicting the properties of a crude oil or its fractions comprising the selection of various physico-chemical parameters, creating a training set with reference samples, analysis of the samples via Gas Chromatography-Mass Spectrometry (GC-MS) for generating coefficients which, when multiplied by the results obtained from the GC-MS analysis of an unknown sample, predict various performance parameters of the crude oil.

This method requires an extremely complex physico-mathematical approach and a high number of reference samples.

In the state of the art, there are also other quality prediction models of hydrocarbons with the use of neural networks. These methods, however, are applied to refined fractions and require data from complex instrumentations (mass spectrometry, NMR, etc.), whereas analogous models which use simple analyses commonly effected on site, have never been successfully applied to crude oils.

American patent US 6,477,516, for example, describes a method for predicting the parameters of hydrocarbons using neural networks. In particular, the above patent describes a method which provides extracting at least one datum from an NMR spectrum of a hydrocarbon to be supplied to a neural network, wherein said neural network is trained to correlate the values extracted from the spectrum with the parameters of the hydrocarbon, so as to be able to predict the desired parameters from the information extracted from the NMR spectrum.

Another method is described by the patent US 5,572,030 for evaluating certain parameters of a hydrocarbon, wherein the infrared spectrum (NIR) of a hydrocarbon is codified and reduced into a certain number of points, which are then supplied to a neural network. This neural network is trained to correlate the points obtained from the spectrum with the desired parameters of the hydrocarbon.

None of the above disclosures describes a method capable of adequately predicting a complete set of yields and qualities of a crude oil using neural networks starting from few easily determinable analytical data, neither has anyone described neural networks optimized and constructed by means of genetic algorithms.

The Applicant has considered the problem of finding a simple and effective method for predicting the qualities and yields of crude oils with the use of neural networks, thus overcoming the above drawbacks.

Artificial neural networks are mathematical models which represent the interconnection between defined elements called neurons. Each neuron or node receives input signals, processes them according to transfer functions and transmits the result to the subsequent nodes. Each node input has a specific weight which serves to quantify its importance. The single nodes are connected to the array of subsequent neurons so as to form a network of neurons. The behaviour of each node is characterized by specific transfer functions. Each connection level between consecutive neurons is commonly indicated with the term layer. The input and output levels, for example, form two layers of the neural network.

The scientific paper Blanco M. et al. "Geographical origin classification of petroleum crudes from near-infrared spectra bitumens", Applied Spectroscopy July 2001, pages 834 to 839, discloses a method of predicting the origin of bitumen based on near-infrared spectroscopy, principal components analysis and cluster analysis. Moreover, the classification results are introduced into an artificial neural network model.

The scientific paper Blanco M. et al. "Determination of physical properties of bitumen by use of near-infrared spectroscopy with neural networks. Joint modeling of linear and non-linear parameters", Analyst, 2001, 126, pages 378 to 382, discloses the use of an artificial neural network model to determine simultaneously two or more non-linear parameters of bitumen.

The Applicant has looked for a rapid system for predicting the quality of crude oils in terms of yields in the various fractions and relative physico-chemical characteristics without requiring the long distillation phase of the crude oil and subsequent characterization of the single fractions, as required by the overall standard analytical procedures envisaged in the crude assay.

The objective of the present invention is to provide a method for predicting the qualities and yields of crude oils by the application of neural networks and genetic algorithms.

An object of the present invention therefore relates to a method for predicting the qualities and yields of crude oils by the application of neural networks and genetic algorithms, characterized in that it comprises the following phases:

a) optimizing a neural network system by means of a genetic algorithm according to input and output values known a priori;
b) determining a sufficient number of physico-chemical characteristics of an unknown crude oil;
c) supplying said physico-chemical characteristics to a neural network system consisting of a neural grouping network and at least one neural prediction network wherein said neural networks are constructed and optimized by means of genetic algorithms;
d) applying said physico-chemical characteristics to said neural grouping network to associate said unknown crude oil with a predefined group to which a specific neural prediction network corresponds;
e) applying said physico-chemical characteristics to said neural prediction network of said predefined group to predict yields and quality parameters of said unknown crude oil.

For the purposes of the present invention, the terms crude oil or unrefined oil indifferently comprise oil as it is extracted from the reservoirs, or straight run fuel oil or a mixture of different crude oils.

The present invention allows the yields or contents of the various fractions present in a crude oil, i.e. gas, naphtha, kerosene, gas oil, vacuum distillate and oily residue, to be predicted.

With reference to the present description and following claims, the definitions of the numerical ranges always comprise the extremes unless otherwise specified.

According to a preferred embodiment of the present invention, said physico-chemical characteristics of said unknown crude oil can be selected from: API gravity, sulfur content, viscosity, acidity, carbon residue (Conradson Carbon Residue R.C.C.), Nickel content, Vanadium content, UOP K factor, asphaltenes, density, smoke point, freezing point, cloud point and pour point.

According to a preferred embodiment, the method according to the present invention is used for predicting the yields of the crude oil in terms of: gas content, naphtha content, kerosene content, gasoil content, vacuum distillate content and residue. In particular, for each of these fractions, the predicted quality parameters can be selected from: density, sulfur content, viscosity, acidity, content of aromatic compounds, paraffins content, naphthenes content, smoke point, freezing point, cloud point and pour point, carbon residue (Conradson Carbon Residue R.C.C.), asphaltenes, Nickel content, Vanadium content.

Some of these parameters can form both the input and output data for the neural network system.

The number of characteristics of the crude oil determined in step a), to be used as input to the grouping network, is conveniently at least 4, preferably from 5 to 7. An expert in the field will determine the number of input characteristics on the basis of the desired level of accuracy of the prediction. This correlation is acquired empirically by an expert on the basis of his experience and the preliminary tests carried out during the training phase of the neural network system according to the present invention.

According to a preferred embodiment of the present invention, said predefined group is a combination of value ranges of said physico-chemical characteristics of the crude oil.

Said neural grouping network selects from the existing groups that which is most representative of the unknown crude oil.

It should be pointed out that the optimum number of said predefined groups depends on the number of types of crude oil used in the training phase, in particular, satisfactory results have been obtained with a number of groups ranging from 5 to 10, preferably ranging from 6 to 8, more preferably equal to 7 groups.

The present invention has the further advantage of providing a method which allows a prediction of the yields and qualities of crude oils with short response times.

The present invention also has the further advantage of providing a method which can be used for predicting the parameters of crude oils without requiring further processings on the sample of crude oil to be analyzed with respect to those necessary for characterizing it.

A further advantage of the present invention is to provide a method which can be used for predicting the quality parameters and yields of a wide range of crude oils including straight run fuel oil, and mixtures of different crude oils.

According to a preferred embodiment of the present invention said neural network system can consist of neural grouping networks of the type known in the art with the name Self Organizing Map (SOM) and neural prediction networks of the type known in the art with the name Back Propagation (BP).

The system of neural networks according to the present invention requires a preliminary training phase to enable it to be used.

Said training phase of the system of neural networks uses a preselected set of physico-chemical characteristics obtained from experimental measurements on different sample crude oils, wherein said characteristics comprise or are the same used as input in said method for predicting the quality and yields of a crude oil.

Said training phase provides a learning process of the neural network system, wherein said networks are optimized by means of genetic algorithms according to input and output values known a priori.

Said neural network system is trained by applying known input and output values, obtained with empirical laboratory analyses or by means of computational methods or known models.

Said system of neural networks allows the prediction of yields and quality parameters of an unknown crude oil after training with physico-chemical characteristics, yields and quality parameters of known crude oils, obtained by means of analytical characterizations.

The training phase comprises the following phases:

> i) applying physico-chemical characteristics of known crude oils as input and the corresponding yields and quality parameters of known crude oils as output, to said neural network system;
> ii) constructing and optimizing said neural grouping network by means of genetic algorithms, distributing the input data in a convenient number of groups, so that each group corresponds to an optimum homogeneity level.
> iii) for each of said groups, constructing and optimizing a neural prediction network associated therewith, using genetic algorithms and attributing the physico-chemical characteristics and corresponding yields and quality parameters of said known crude oils relating to said group;
> iv) validating said neural network system by minimization of the deviation between the values of the predicted yields and quality parameters and the corresponding values of the known crude oils.

Advantageously said optimum homogeneity level corresponds to implicit criteria in self-organizing map algorithms, for example the Kohonen algorithm.

In particular, the above neural grouping network of the SOM type subdivides the data into different groups minimizing the Euclidean distance of each value of said physico-chemical characteristics from the relative average datum of the group.

In a preferred embodiment of the present invention, said number of groups is conveniently selected by an expert on the basis of his own experience or by carrying out preliminary processing tests which minimize the deviation between the experimental data and the data calculated by means of said neural network system.

According to a particular embodiment of the method, the training phase of said neural prediction network can be effected with known samples belonging to said group and with known samples whose physico-chemical characteristics are very close to the limit values of the ranges which define said group (for example +/-5% of the limit values of the range).

Said training phase envisages the construction for each predefined group of a predictive neural network, whose input data can be the same as the neural grouping network.

It should also be noted that the groups are selected on the basis of the most effective inputs in describing the system and in minimizing the error.

According to a preferred embodiment of the present invention, there are at least 30 of said types of known crude oils, preferably at least 100, more preferably from 100 to 500.

It should be pointed out that self-organizing maps are neural networks with a single layer wherein the outgoing neurons are organized in grids. Each input is connected to all the outgoing neurons and each neuron is associated with the weight vector having the same dimensions as the incoming vectors.

The dimension of the incoming vector is generally much higher than the dimension of the outgoing grid, for this reason SOM neural networks are mainly used for the reduction of the dimension rather than the expansion.

According to a preferred embodiment of the present invention, the transfer functions of the neurons of said neural prediction network of the back propagation type can be linear or alternatively hyperbolic tangents.

According to a preferred embodiment of the present invention, the number of layers of each neural network can variably depend on the type of crude oil analyzed. In particular, it has been found that the method according to the present invention offers good results with at least 3 layers comprising the input and output layers, said number of layers conveniently ranges from 4 to 5.

According to a preferred embodiment of the present invention, during the training process of the neural network system, randomly selected initial weights are adjusted and optimized after each start-up or period (iteration process) until the correlation coefficient "r" between the known values and predicted values is maximized.

It should be pointed out that the correlation coefficient "r" should be considered as being:

$$r = \frac{\text{variations of Y associated with variations of X}}{\text{independent variations of Y with respect to variations of X}}$$

$$= \frac{\text{co-variability of X and Y}}{\text{variability of X + variability of Y}}$$

wherein Y represents the predicted value and X is the known value.

Alternatively, said weights are adjusted and optimized until the prediction error is minimized, preferably the average quadratic error or absolute error.

It should be noted that the average quadratic error $\sigma$ of a distribution should be considered as being the quadratic average of the rejects of the single data from their arithmetical average M.

$$\sigma = \sqrt{\frac{\sum_{i=1}^{n} (x_i - M)^2}{n}}$$

wherein $x_1$, $x_2$, ..., $x_n$ represent the predicted data and M the arithmetical average of the known data.

It should also be pointed out that the absolute error of a datum should be considered as:

$$|E_{abs}| = |V_{pred} - V_{known}|$$

wherein $E_{abs}$ represents the absolute value, $V_{pred}$ the predicted value and $V_{known}$ the known value.

Furthermore, it should be noted that the transfer functions of the nodes of the neural network are improved with the training so that the response obtained deviates less and less from the effective response.

It should be pointed out, moreover, that the neural network trained according to the present invention can be developed with the use of any specific software available on the market or any other system known to experts in the field.

The data calculated can also be used as input for Linear Programming (LP) software, used for optimizing industrial refining processes.

It should be noted that the neural network can be accurately tested with various known sample parameters, for verifying that the result falls within standards held as being acceptable.

According to a preferred embodiment of the present invention, said neural networks in the training phase are optimized and constructed with the help of genetic algorithms according to the following steps:

1. creating an initial population of genotypes (genetic representations of neural networks);
2. constructing phenotype neural networks based on the genotypes;
3. training and testing the neural networks to determine to what extent they correspond to the discriminating parameter;
4. comparing the results obtained to select the best networks;
5. selecting the best networks between the populations available and discarding the worst;

6. bringing the population selected back to a preestablished calculation value according to one of the following sub-phases:

> 6.1 intercrossing the genotypes of the neural networks;
> 6.2 coupling the genotypes by the exchange of genes (characteristics) between the networks;
> 6.3 changing the genotypes according to a random modality;

7. resuming the process from step 2 until reaching a stop criterion.

Said stop criterion, as also that for selecting the best neural networks, can be the maximization of the correlation coefficient "r" between known values and those predicted by the system or alternatively minimization of the error, preferably the average quadratic error or absolute error, preferably avoiding the phenomenon known in the state of the art as "over learning".

Through this construction and optimization phase, the best networks survive, their characteristics are carried forward into future generations and combined with others so as to obtain increasingly improved networks for the specific application.

The genetic combination process of the factors considerably improves the identification velocity of the most suitable networks, as the properties of genetic research are much more effective than those of random research.

This method advantageously allows new and better solutions to be obtained with respect to those that can be obtained experimentally by the user.

Further characteristics and advantages of the method for predicting the quality and yields of crude oils by the application of neural networks and genetic algorithms will appear more evident from the following description of one of its embodiments, provided purely for illustrative and non-limiting purposes, with reference to the enclosed drawings, in which:

- figure 1 schematically illustrates the training procedure of the neural grouping network;
- figure 2 schematically illustrates the training procedure of the neural prediction network;
- figure 3 schematically illustrates the calculation procedure to be applied to an unknown sample using the neural grouping and prediction networks.

With reference to figure 1, this illustrates a preferred training method of the neural grouping network, comprising a definition phase of the samples to be analyzed (101), wherein different types of crude oil are selected and then characterized by means of standard analytical methods (102).

A series of physico-chemical parameters relating to the samples characterized (103) are used as input data for the training phase.

The neural grouping network is trained (104) to group said crude oils into a number of groups preselected by an expert in the field on the basis of his experience and possible preliminary calculations/tests.

The neural network is subjected to an error validation process according to a predefined standard (105).

If this is not reached, a subsequent optimization phase is effected by means of genetic algorithms (106) until the prefixed standard is reached.

This standard is normally based on a data homogeneity criterion.

When the prefixed standard is reached, the neural grouping network is ready (108) for the subsequent prediction phase.

With reference to figure 2, once the neural grouping network is ready, the same number of neural prediction networks as the groupings must be created and optimized.

For each generic group k (201), the physico-chemical parameters of the known crude oils belonging to the group, together with the yields and qualities of the same (202), are used as training input of the corresponding prediction network.

Each neural network is trained to predict the yields and qualities (204) and the predicted parameters are then compared with the known parameters (206).

If the above comparison does not correspond to a prefixed validation standard, said neural network is optimized by means of genetic algorithms (205) and is subjected again to the training phase (204).

The training and identification phase of the neural prediction network ends (207) when the prefixed validation standard is reached.

This standard can be the error minimization or alternatively the maximization of the correlation coefficient "r" between the predicted parameters and the known parameters.

With reference to figure 3, the neural network system thus constructed and optimized according to the training procedure described above, can be used for predicting the characteristics of an unknown sample (301).

By acquiring the characteristics of the crude oil corresponding to the prefixed parameters during the training of the neural network system by means of standard laboratory analysis (302), the data to be imputed at the inlet of the neural network system, are defined.

The neural grouping network allows the unknown sample to be directed towards the most appropriate predefined group (303).

A subsequent neural prediction network associated with the group relating to the unknown crude oil, allows the yields and qualities of same to be predicted (304).

An illustrative and non-limiting example of the present invention is provided hereunder for a better understanding of the same and for its embodiment.

Example 1 - Training phase

The statistical databank used for training the system of neural networks was created with different types of crude oils coming from various geographical areas.

A large number of crude oils was used for training the neural network system on numerous processings (over 400 crude oils), using neural networks optimized by means of genetic algorithms. The geographical distribution of the databank is indicated in Table 1.

Table 1

| AREA | % with respect to total | Max di °API | Min di °API | Maximum sulfur content % | Minimum sulfur content % |
|---|---|---|---|---|---|
| Black Sea | 8.76% | 36.8 | 26.2 | 2.78 | 0.30 |
| Caspian Sea | 2.43% | 50.7 | 26.9 | 1.29 | 0.14 |
| Far East | 0.49% | 40.0 | 38.9 | 0.04 | 0.04 |
| Middle East | 22.38% | 50.0 | 15.8 | 8.19 | 0.02 |
| North Africa | 25.30% | 47.2 | 19.9 | 4.02 | 0.01 |
| North Sea | 7.06% | 45.2 | 18.9 | 1.32 | 0.16 |
| South America | 5.11% | 38.4 | 10.9 | 7.40 | 0.08 |
| West Africa | 13.38% | 48.5 | 17.3 | 2.08 | 0.03 |
| Mediterranean Europe | 15.09% | 43.4 | 9.4 | 7.15 | 0.12 |
| TOTAL | 100.00% | 50.7 | 9.4 | 8.19 | 0.01 |

The crude oils were analytically characterized in terms of density, sulfur content, viscosity, acidity (Total Acid Number, TAN), Nickel (Ni) content, Vanadium (V) content, UOP K factor, C7 Asphaltenes insoluble in n-heptane, Conradson Carbon Residue (CCR), TBP (True Boiling Point) distillation yields and physico-chemical characteristics of the TBP fractions.

The quality ranges of the crude oils considered in the statistical analyses are indicated in Table 2.

Table 2

| | Min | Max |
|---|---|---|
| °API | 9.4 | 50.68 |
| Sulfur (weight %) | 0.01 | 8.19 |
| Viscosity at 20°C (cSt) | 1.319 | 31.242 |
| TAN (mgKOH/g) | 0 | 3.5 |
| Ni (ppm) | 0 | 131.1 |
| V (Ppm) | 0 | 583.5 |
| UOP K factor | 11.1 | 12.9 |

(continued)

|  | Min | Max |
|---|---|---|
| nC7 asphaltenes (weight %) | 0 | 20.45 |
| CCR (weight %) | 0.1 | 20.64 |

The training phase of the grouping network of the SOM type was established on the basis of 7 groups.

The physico-chemical characteristics used are those which proved to be most effective in dividing the crude oils into homogeneous groups, and corresponding to analytical methods which appear to be the most rapid and easily accessible: density, API gravity, sulfur content, viscosity at 20°C, Ni content, V content, Conradson Carbon Residue (CCR), acidity (Total Acid Number, TAN), UOP K factor, C7 Asphaltenes insoluble in n-heptane.

Neural prediction networks of the back propagation type were trained and optimized for each of the 7 groups obtained, with different architectures for each of the groups: 4 layers including input and output layers, different numbers of nodes and different transfer functions.

The groups obtained and relative ranges of the characteristics are indicated in Table 3.

Table 3.

| Groups | °API | | | Sulfur (weight %) | | | Viscosity at 20°C (cSt) | TAN (mgKOH/ g) | Ni (ppm) | V (ppm) | UOPK factor | Asphaltenes nC7 (wt %) | CCR(wt %) |
|---|---|---|---|---|---|---|---|---|---|---|---|---|---|
| | average | MIN | MAX | average | MIN | MAX | | | | | | | |
| 1 | 19.30 | 9.40 | 25.50 | 4.07 | 1.32 | 8.19 | 2766 | 0.92 | 49.8 | 126.6 | 11.6 | 8.26 | 10.50 |
| 2 | 28.24 | 25.10 | 31.90 | 2.29 | 1.80 | 3.11 | 68.64 | 0.22 | 22.4 | 40.0 | 11.8 | 2.82 | 5.96 |
| 3 | 33.34 | 29.77 | 40.00 | 2.13 | 1.77 | 2.80 | 11.42 | 0.12 | 10.9 | 28.8 | 11.9 | 1.61 | 4.62 |
| 4 | 32.03 | 24.00 | 36.90 | 1.36 | 0.78 | 1.79 | 21.79 | 0.25 | 19.7 | 45.4 | 11.9 | 1.53 | 4.22 |
| 5 | 26.50 | 17.30 | 32.41 | 0.30 | 0.12 | 0.75 | 330.7 | 0.91 | 14.6 | 5.3 | 11.8 | 0.85 | 4.26 |
| 6 | 36.70 | 32.60 | 40.40 | 0.36 | 0.04 | 1.40 | 9.302 | 0.13 | 4.6 | 4.3 | 12.0 | 0.51 | 2.34 |
| 7 | 44.21 | 40.50 | 50.68 | 0.19 | 0.01 | 0.82 | 3.780 | 0.17 | 1.3 | 1.1 | 12.1 | 0.18 | 0.97 |

A summarizing scheme of the input data, yields of the cut and relative qualitative parameters which can be predicted with the neural network (output) is indicated in Table 4.

Table 4

| | | CRUDE OIL | YIELDS AND CHARACTERISTICS OF THE PRODUCTS | | | | | | |
|---|---|---|---|---|---|---|---|---|---|
| | | | GAS | NAPHTHAS | | KEROSENES | GAS OILS | VACUUM DISTILLATE | OILY RESIDUES |
| Boiling range : | °C | | C1-C4 | C5-80 | 80-160 | 160-230 | 230-370 | 370-530 | 530+ |
| Yields | (weight %) | | output | output | output | output | output | output | output |
| Density at 15°C | Kg/l | | | output | output | output | output | output | output |
| °API | | input | | | | | | | |
| Viscosity at 20°C | cSt | input | | | | | | | |
| Viscosity at 50°C | VBN | | | | | output | output | output | output |
| Sulfur | (weight %) | input | | | | output | output | output | output |
| Acidity | mgKOH/g | input | | | | | | | |
| Paraffins | (volume %) | | | | output | | | | |
| Naphthenes | (volume %) | | | | output | | | | |
| Aromatics | (volume %) | | | | output | | | | |
| N+2° | (volume %) | | | | output | | | | |
| Smoke point | mm | | | | | output | | | |
| Freezing point | °C | | | | | output | | | |
| Cloud point | °C | | | | | | output | | |
| Pour point | °C | | | | | | output | | |
| Nickel | ppm | input | | | | | | | output |
| Vanadium | ppm | input | | | | | | | output |
| Asphthaltenes in NC7 | (weight %) | output | | | | | | | output |
| R.C.C. | (weight %) | input | | | | | | output | output |

EP 2 416 153 B1

Tables 5-8 indicate the consistency data on the TBP (True Boiling Point) yields and on the properties of the TBP key fractions obtained from trained and optimized neural networks with respect to each group on the basis of known crude oils.

The average absolute errors are expressed as:

$$\left| \sum_n \frac{Calculated\_value - known\_value}{n} \right|$$

Table 5

|  |  | GROUP | 1 | 2 | 3 | 4 | 5 | 6 | 7 | Total |
|---|---|---|---|---|---|---|---|---|---|---|
| Error on fraction obtained in wt% | C1-C4 Fraction | Absolute error | 0.042 | 0.11 | 0.163 | 0.117 | 0.046 | 0.133 | 0.158 | 0.118 |
|  | C5-80 Fraction | Absolute error | 0.195 | 0.2 | 0.284 | 0.238 | 0.093 | 0.217 | 0.138 | 0.207 |
|  |  | % of results with $\Delta < 1$ (*) | 98.11 | 100 | 97.22 | 98.57 | 100 | 98.13 | 98.31 | 98.51 |
|  |  | % of results with $\Delta < 2$ (*) | 100 | 100 | 98.61 | 100 | 100 | 98.13 | 100 | 99.36 |
|  | 80-160 Fraction | Absolute error | 0.22 | 0.265 | 0.337 | 0.306 | 0.137 | 0.393 | 0.303 | 0.302 |
|  |  | % of results with $\Delta < 1$ (*) | 98.11 | 100 | 95.83 | 97.14 | 100 | 95.33 | 93.22 | 96.81 |
|  |  | % of results with $\Delta < 2$ (*) | 100 | 100 | 100 | 100 | 100 | 97.2 | 98.31 | 99.15 |
|  | 160-230 Fraction | Absolute error | 0.16 | 0.15 | 0.227 | 0.194 | 0.129 | 0.265 | 0.13 | 0.193 |
|  |  | % of results with $\Delta < 1$ (*) | 100 | 100 | 97.22 | 98.57 | 100 | 96.26 | 98.31 | 98.30 |
|  |  | % of results with $\Delta < 2$ (*) | 100 | 100 | 100 | 100 | 100 | 99.07 | 100 | 99.79 |

**EP 2 416 153 B1**

(continued)

| | | GROUP | 1 | 2 | 3 | 4 | 5 | 6 | 7 | Total |
|---|---|---|---|---|---|---|---|---|---|---|
| | 230-370 Fraction | Absolute error | 0.476 | 0.267 | 0.28 | 0.239 | 0.18 | 0.431 | 0.279 | 0.322 |
| | | % of results with $\Delta$ <1 (*) | 88.68 | 98.51 | 97.22 | 100 | 97.62 | 96.26 | 91.53 | 95.96 |
| | | % of results with $\Delta$ <2 (*) | 94.34 | 100 | 100 | 100 | 100 | 97.2 | 100 | 98.72 |
| | 370-530 Fraction | Absolute error | 0.321 | 0.471 | 0.387 | 0.383 | 0.286 | 0.355 | 0.308 | 0.367 |
| | | % of results with $\Delta$ <1 (*) | 96.23 | 88.06 | 94.44 | 95.71 | 90.48 | 93.46 | 93.22 | 93.19 |
| | | % of results with $\Delta$ <2 (*) | 100 | 97.01 | 100 | 98.57 | 97.62 | 96.26 | 100 | 98.30 |
| | 530+ Fraction | Absolute error | 0.402 | 0.478 | 0.483 | 0.428 | 0.297 | 0.444 | 0.297 | 0.42 |
| | | % of results with $\Delta$ <1 (*) | 90.57 | 88.06 | 91.67 | 90 | 92.86 | 92.52 | 94.92 | 91.49 |
| | | % of results with $\Delta$ <2 (*) | 98.11 | 97.01 | 98.61 | 98.57 | 97.62 | 98.13 | 94.92 | 97.66 |
| (*) $\Delta$ = |experimental datum -calculated datum| | | | | | | | | | | |

Table 6

| | | GROUP | 1 | 2 | 3 | 4 | 5 | 6 | 7 | TOTAL |
|---|---|---|---|---|---|---|---|---|---|---|
| | 160-230 Fraction | Absolute error | 0.055 | 0.023 | 0.016 | 0.012 | 0.004 | 0.004 | 0.004 | 0.015 |
| | | % results with error <15% | 73.58 | 74.63 | 80.56 | 71.43 | 80.95 | 52.34 | 33.9 | 65.32 |
| | | % results with error <20% | 79.25 | 80.6 | 88.89 | 80 | 88.1 | 61.68 | 38.98 | 72.77 |

(continued)

| | | GROUP | 1 | 2 | 3 | 4 | 5 | 6 | 7 | TOTAL |
|---|---|---|---|---|---|---|---|---|---|---|
| Error in wt % of sulfur | 230-370 Fraction | Absolute error | 0.082 | 0.041 | 0.063 | 0.029 | 0.004 | 0.015 | 0.008 | 0.034 |
| | | % results with error <15% | 94.34 | 98.51 | 94.44 | 98.57 | 100 | 75.7 | 83.05 | 90.43 |
| | | % results with error <20% | 96.23 | 98.51 | 95.83 | 100 | 100 | 85.98 | 89.83 | 94.26 |
| | 370-530 Fraction | Absolute error | 0.08 | 0.041 | 0.084 | 0.037 | 0.006 | 0.018 | 0.012 | 0.039 |
| | | % results with error <15% | 98.11 | 100 | 98.61 | 100 | 100 | 89.72 | 91.53 | 96.17 |
| | | % results with error <20% | 100 | 100 | 98.61 | 100 | 100 | 92.52 | 96.61 | 97.66 |
| | 530+ Fraction | Absolute error | 0.119 | 0.074 | 0.166 | 0.064 | 0.012 | 0.032 | 0.025 | 0.07 |
| | | % results with error <15% | 100 | 100 | 97.22 | 100 | 100 | 92.52 | 89.83 | 96.6 |
| | | % results with error <20% | 100 | 100 | 98.61 | 100 | 100 | 94.39 | 93.22 | 97.66 |
| (*) Δ = \|experimental datum -calculated datum\| | | | | | | | | | | |

Table 7

| | | GROUP | 1 | 2 | 3 | 4 | 5 | 6 | 7 | TOTAL |
|---|---|---|---|---|---|---|---|---|---|---|
| Error on N2A (volume % Naphthenes + 2*volume % Aromatics) on the 80-160 fraction | | Absolute error | 1.568 | 1.025 | 1.455 | 0.881 | 1.023 | 1.427 | 0.74 | 1.186 |
| | | % of results with Δ <2 (*) | 83.02 | 83.58 | 83.33 | 95.71 | 85.71 | 86.92 | 93.22 | 87.45 |
| | | % of results with Δ <4 (*) | 92.45 | 98.51 | 93.06 | 98.57 | 97.62 | 95.33 | 96.61 | 95.96 |

(continued)

| | GROUP | 1 | 2 | 3 | 4 | 5 | 6 | 7 | TOTAL |
|---|---|---|---|---|---|---|---|---|---|
| Error Freezing Point in °C on the 160-230 fraction | Absolute error | 1.002 | 1.454 | 0.895 | 1.147 | 1.497 | 0.901 | 0.547 | 1.036 |
| | to of results with Δ <2 (*) | 86.79 | 77.61 | 91.67 | 84.29 | 73.81 | 90.65 | 93.22 | 86.38 |
| | % of results with Δ <4 (*) | 96.23 | 91.04 | 98.61 | 97.14 | 95.24 | 96.26 | 98.31 | 96.17 |
| Error Pour Point in °C on the 230-370 fraction | Absolute error | 1.322 | 0.698 | 0.699 | 0.783 | 0.7 | 0.665 | 0.539 | 0.764 |
| | % of results with Δ <3 (*) | 90.57 | 98.51 | 97.22 | 97.14 | 100 | 98.13 | 94.92 | 96.81 |
| | % of results with Δ <6 (*) | 94.34 | 98.51 | 100 | 100 | 100 | 100 | 100 | 99.15 |
| (*) Δ = |experimental datum -calculated datum| | | | | | | | | | |

Table 8

| | GROUP | 1 | 2 | 3 | 4 | 5 | 6 | 7 | TOTAL |
|---|---|---|---|---|---|---|---|---|---|
| Error on viscosity at 50°C on the 370-530 fraction | Absolute error | 0.138 | 0.145 | 0.167 | 0.275 | 0.136 | 0.355 | 0.174 | 0.218 |
| | % of results with Δ <0.5 (*) | 96.23 | 95.52 | 91.67 | 88.57 | 95.24 | 75.7 | 93.22 | 89.15 |
| | % of results with Δ <2 (*) | 98.11 | 100 | 98.61 | 97.14 | 97.62 | 94.39 | 94.92 | 97.02 |
| Error on viscosity at 50°C on the 530+ fraction | Absolute error | 0.638 | 0.217 | 0.29 | 0.28 | 0.333 | 0.407 | 0.332 | 0.353 |
| | % of results with Δ <1 (*) | 69.81 | 89.55 | 86.11 | 84.29 | 83.33 | 75.7 | 84.75 | 81.7 |
| | % of results with Δ <2 (*) | 84.91 | 98.51 | 98.61 | 97.14 | 88.1 | 91.59 | 91.53 | 93.4 |
| Error on wt % of asphaltenes nC7 on the 530+ fraction | Absolute error | 0.706 | 0.612 | 0.719 | 0.755 | 0.12 | 0.505 | 0.317 | 0.555 |
| | % of results with error <20% | 98.11 | 94.03 | 76.39 | 65.71 | 69.05 | 63.55 | 62.71 | 74.47 |
| | % of results with error <30% | 98.11 | 95.52 | 91.67 | 88.57 | 95.24 | 76.64 | 76.27 | 87.45 |

(continued)

| | GROUP | 1 | 2 | 3 | 4 | 5 | 6 | 7 | TOTAL |
|---|---|---|---|---|---|---|---|---|---|
| Error on wt % CCR on the 530+ fraction | Absolute error | 0.31 | 0.404 | 0.503 | 0.293 | 0.166 | 0.418 | 0.297 | 0.36 |
| | % of results with error <15% | 100 | 98.51 | 100 | 100 | 100 | 98.13 | 94.92 | 98.72 |
| | % of results with error <20% | 100 | 98.51 | 100 | 100 | 100 | 99.07 | 94.92 | 98.94 |
| (*) Δ = \|experimental datum - calculated datum\| | | | | | | | | | |

As can be seen from Tables 5-8, the neural network system according to the present invention allows results having a high accuracy and reliability to be obtained after training.

EXAMPLE 2

The neural network system trained according to the previous example was used for predicting yields and qualities of an unknown crude oil "A" coming from the Black Sea.
Table 9 indicates the input characteristics measured on the crude oil and predicted yields for the various fractions, in addition, for comparative purposes, to the corresponding values obtained experimentally from distillation of the crude oil and characterization of the fractions.

Tabella 9

| | | Crude oil (INPUTS) | YIELDS AND CHARACTERISTICS OF THE PRODUCTS | | | | | | | | | | | | |
|---|---|---|---|---|---|---|---|---|---|---|---|---|---|---|---|
| | | | GAS | | NAPHTHAS | | | | KEROSENES | | GAS OILS | | VACUUM DISTIL. | | OILY RESIDUES | |
| TBP range: | | | C1-C4 | | C5-80 | | 80-160 | | 160-230 | | 230-370 | | 370-530 | | 530+ | |
| | | | known | calculated | known | calculated | known | calculated | known | calculated | known | calculated | known | calculated | known | calculated |
| TBP Yield | (peso %) | | 1.13 | 1.51 | 4.06 | 3.80 | 10.82 | 10.25 | 10.40 | 10.74 | 25.43 | 25.63 | 23.24 | 23.60 | 24.93 | 24.29 |
| | | | | | | | | | | | | | | | | |
| Density at 15°C | Kg/l | | 0.5673 | | 0.6706 | 0.6637 | 0.7493 | 0.7449 | 0.8022 | 0.7977 | 0.8583 | 0.8549 | 0.9136 | 0.9135 | 1.0011 | 0.9975 |
| °API at 60°F | | 31.56 | | | | | | | | | | | | | | |
| Viscosity at 20°C | cSt | 16.91 | | | | | | | | | | | | | | |
| Viscosity at 50°C | VBN | | | | | | | | 3.20 | 3.63 | 16.36 | 15.92 | 29.78 | 29.46 | 44.61 | 44.70 |
| Sulfur | (weight %) | 1.36 | | | | | | | 0.18 | 0.18 | 1.04 | 0.96 | 1.57 | 1.69 | 2.73 | 2.94 |
| Acidity | mgKOH/g | 0.19 | | | | | | | | | | | 0.29 | | | |
| Paraffins | (volume %) | | | | | | 50.42 | 56.82 | | | | | | | | |
| N+2A | | | | | | | 58.86 | 50.58 | | | | | | | | |
| Smoke point | mm | | | | | | | | 25 | 25 | | | | | | |
| Freezing point | °C | | | | | | | | -56 | -56 | | | | | | |
| Cloud point | °C | | | | | | | | | | -8 | -5 | | | | |
| Pour point | °C | | | | | | | | | | -9 | -8 | | | | |
| Nickel | ppm | 16 | | | | | | | | | | | | | 64 | 66 |
| Vanadium | ppm | 48 | | | | | | | | | | | | | 193 | 199 |

EP 2 416 153 B1

(continued)

| | | Crude oil (INPUTS) | YIELDS AND CHARACTERISTICS OF THE PRODUCTS | | | | | | | | | | | | | |
| | | | GAS | | NAPHTHAS | | | | KEROSENES | | GAS OILS | | VACUUM DISTIL. | | OILY RESIDUES | |
| TBP range: | | | C1-C4 | | C5-80 | | 80-160 | | 160-230 | | 230-370 | | 370-530 | | 530+ | |
| | | | known | calculated | known | calculated | known | calculated | known | calculated | known | calculated | known | calculated | known | calculated |
| As-phaltenes in NC7 | (weight %) | 0.95 | | | | | | | | | | | | | 3.80 | 5 |
| R.C.C. | (weight %) | 3.85 | | | | | | | | | | | 0.25 | 0.29 | 15.23 | 15.89 |
| UOP K Factor | | 11.9 | | | | | | | | | | | | | | |

EP 2 416 153 B1

EXAMPLE 3

The neural network system trained according to the previous example was used for predicting yields and qualities of an unknown crude oil "B" coming from the North Africa.

Table 10 indicates the input characteristics measured on the crude oil and predicted yields for the various fractions, in addition, for comparative purposes, to the corresponding values obtained experimentally from distillation of the crude oil and characterization of the fractions.

Table 10

| | | Crude oil (INPUTS) | YIELDS AND CHARACTERISTICS OF THE PRODUCTS | | | | | | | | | | | | | |
| --- | --- | --- | --- | --- | --- | --- | --- | --- | --- | --- | --- | --- | --- | --- | --- | --- |
| | | | GAS | | NAPHTHAS | | | | KEROSENES | | GAS OILS | | VACUUM DISTILLATE | | OILY RESIDUES | |
| TBP range: | | | C1-C4 | | C5-80 | | 80-160 | | 160-230 | | 230-370 | | 370-530 | | C1-C4 | |
| | | | known | calculated | known | calculated | known | calculated | known | calculated | known | calculated | known | calculated | known | calculated |
| TBP yield | (weight %) | | 0.95 | 0.99 | 3.38 | 3.83 | 11.23 | 11.39 | 10.55 | 10.29 | 25.48 | 24.99 | 24.11 | 24.36 | 24.29 | 24.27966 |
| Density at 15°C | Kg/l | | 0.5588 | | 0.6617 | 0.6701 | 0.7396 | 0.7406 | 0.7834 | 0.7835 | 0.8276 | 0.8266 | 0.8745 | 0.8713 | 0.9466 | 0.942824 |
| °API a 60°F | | 37.31 | | | | | | | | | | | | | | |
| Viscosity at 20°C | cSt | 17.66 | | | | | | | | | | | | | | |
| Viscosity at 50°C | VBN | | | | | | | | 2.54 | 3.,86 | 15.59 | 15.29 | 27.60 | 27.81 | 40.87 | 41.59943 |
| Sulfur | (weight %) | 0.12 | | | | | | | 0.04 | 0.03 | 0.09 | 0.09 | 0.15 | 0.13 | 0.27 | 0.26 |
| Acidity | mgKOH/g | 0.12 | | | | | | | | | | | | | | |
| Paraffins | (volume %) | | | | | | 56.47 | 60.75 | | | | | | | | |
| N+2A | | | | | | | 51.63 | 45.46 | | | | | | | | |
| Smoke point | mm | | | | | | | | 32 | 30 | | | | | | |
| Freezing point | °C | | | | | | | | -47 | -49 | | | | | | |
| Cloud point | °C | | | | | | | | | | +3 | +5 | | | | |
| Pour point | °C | | | | | | | | | | +0 | +3 | | | | |
| Nickel | ppm | 3 | | | | | | | | | | | | | 14 | 14 |
| Vanadium | ppm | 1 | | | | | | | | | | | | | 4 | 3 |

EP 2 416 153 B1

(continued)

| | | Crude oil (INPUTS) | YIELDS AND CHARACTERISTICS OF THE PRODUCTS | | | | | | | | | |
|---|---|---|---|---|---|---|---|---|---|---|---|---|
| | | | GAS | | NAPHTHAS | | KEROSENES | | GAS OILS | | VACUUM DISTILLATE | | OILY RESIDUES |
| TBP range: | | | C1-C4 | | C5-80 | 80-160 | 160-230 | | 230-370 | | 370-530 | | C1-C4 |
| As-phaltenes in NC7 | (weight %) | | | | | | | | | | | 0.55 | 1 |
| R.C.C. | (weight %) | 2.54 | | | | | | | | | 0.07 | 0.06 | 10.40 | 10.43 |
| UOP K Factor | | | | | | | | | | | | | |

**Claims**

1. A method for predicting the quality and yields of a crude oil by the application of neural networks and genetic algorithms, **characterized in that** it comprises the following phases:

   a) optimizing a neural network system by means of a genetic algorithm according to input and output values known a priori;
   b) determining a sufficient number of physico-chemical characteristics of an unknown crude oil;
   c) supplying said physico-chemical characteristics to a neural network system consisting of a neural grouping network and at least one neural prediction network wherein said neural networks are constructed and optimized by means of genetic algorithms;
   d) applying said physico-chemical characteristics to said neural grouping network to associate said unknown crude oil with a predefined group to which a specific neural prediction network corresponds;
   e) applying said physico-chemical characteristics to said neural prediction network of said predefined group to predict yields and quality parameters of said unknown crude oil.

2. The method for predicting the quality and yields of a crude oil according to claim 1, wherein said physico-chemical characteristics of said unknown crude oil are selected from: API gravity, sulfur content, viscosity, acidity, carbon residue, Nickel content, Vanadium content, UOP K factor, asphaltenes, density, smoke point, freezing point, cloud point and pour point.

3. The method for predicting the quality and yields of a crude oil according to claim 1, wherein the predicted yields for the fractions of crude oil refer to: gas content, naphtha content, kerosene content, gasoil content, vacuum distillate content and residue.

4. The method according to claim 3, wherein for each of said fractions, the predicted quality parameters are selected from: density, sulfur content, viscosity, acidity, content of aromatic compounds, paraffin content, naphthene content, smoke point, freezing point, cloud point and pour point, carbonresidue, asphaltenes, Nickel content, Vanadium content.

5. The method for predicting the quality and yields of a crude oil according to claim 1, wherein the number of characteristics of the crude oil determined in step a) is at least 4.

6. The method for predicting the quality and yields of a crude oil according to claim 1, wherein said predefined group is a combination of value ranges of said physico-chemical characteristics of the crude oil.

7. The method for predicting the quality and yields of a crude oil according to claim 1, wherein the number of said predefined groups ranges from 5 to 10.

8. The method for predicting the quality and yields of a crude oil according to claim 1, wherein said neural network system consists of neural grouping networks of the Self Organizing Map type.

9. The method for predicting the quality and yields of a crude oil according to claim 1, wherein said neural network system consists of neural prediction networks of the Back Propagation type.

10. The method for predicting the quality and yields of a crude oil according to any of the previous claims, wherein the transfer functions of the neurons of said neural prediction network of the back propagation type are linear or alternatively hyperbolic tangents.

11. The method for predicting the quality and yields of a crude oil according to any of the previous claims, wherein said neural networks have at least 3 layers, comprising the input and output layers.

12. The method for predicting the quality and yields of a crude oil according to claim 1, also comprising a preliminary training phase.

13. The method for predicting the quality and yields of a crude oil according to claim 12, wherein said training phase comprises the following phases:

i) applying physico-chemical characteristics of known crude oils as input and the corresponding yields and quality parameters of known crude oils as output to said neural network system;

ii) constructing and optimizing said neural grouping network by means of genetic algorithms, distributing the input data in a convenient number of groups, so that each group corresponds to an optimum homogeneity level.

iii) for each of said groups, constructing and optimizing a neural prediction network associated therewith, using genetic algorithms and attributing the physico-chemical characteristics and corresponding yields and quality parameters of said known crude oils relating to said group;

iv) validating said neural network system by minimization of the deviation between the values of the predicted yields and quality parameters and the corresponding values of the known crude oils.

14. The method for predicting the quality and yields of a crude oil according to claim 13, wherein said optimum homogeneity level corresponds to the Kohonen algorithm.

15. The method for predicting the quality and yields of a crude oil according to claim 13, wherein the input data of said neural prediction network are the same as the neural grouping network.

16. The method for predicting the quality and yields of a crude oil according to claim 13, wherein there are at least 30 of said types of known crude oils.

17. The method for predicting the quality and yields of a crude oil according to claim 13, wherein during said training phase, randomly selected initial weights are assigned to said neural networks, which are subsequently adjusted and optimized until the "r" correlation coefficient between the known values and predicted values is maximized.

18. The method for predicting the quality and yields of a crude oil according to claim 13, wherein during said training phase, randomly selected initial weights are assigned to said neural networks, which are subsequently adjusted and optimized until the prediction error is minimized.

19. The method for predicting the quality and yields of a crude oil according to claim 13, wherein said neural networks in the training phase are optimized and constructed with the help of genetic algorithms according to the following steps:

1. creating an initial population of genotypes;
2. constructing phenotype neural networks based on the genotypes;
3. training and testing the neural networks to determine to what extent they correspond to the discriminating parameter;
4. comparing the results obtained to select the best networks;
5. selecting the best networks from the populations available and discarding the worst;
6. bringing the population selected back to a pre-established calculation value according to one of the following sub-phases:

   - intercrossing the genotypes of the neural networks;
   - coupling the genotypes by the exchange of genes between the networks;
   - changing the genotypes according to a random modality;

7. resuming the process from step 2 until reaching a stop criterion.

20. The method for predicting the quality and yields of a crude oil according to claim 19, wherein said stop criterion is the maximization of the "r" correlation coefficient between the known values and those predicted by the system or alternatively the minimization of the error.

**Patentansprüche**

1. Verfahren zur Vorhersage der Rohölqualität und Rohölausbeute durch Anwendung neuronaler Netzwerke und genetischer Algorithmen, **dadurch gekennzeichnet dass** es die folgenden Phasen umfasst:

a) Optimieren eines neuronalen Netzwerksystems mittels eines genetischen Algorithmus gemäß Eingabe- und Ausgabewerten die a priori bekannt sind;

b) Bestimmen einer ausreichenden Anzahl von physiko-chemischen Eigenschaften eines unbekannten Rohöls;

c) Bereitstellen der physiko-chemischen Eigenschaften an ein neuronales Netzwerksystem bestehend aus einem neuronalen Gruppierungsnetzwerk und mindestens einem neuronalen Vorhersagenetzwerk, wobei die neuronalen Netzwerke mittels genetischer Algorithmen konstruiert und optimiert sind;

d) Anwenden der physiko-chemischen Eigenschaften auf das genannte neuronale Gruppierungsnetzwerk, um das unbekannte Rohöl mit einer vordefinierten Gruppe zu verknüpfen, welcher ein spezifisches neuronales Vorhersagenetzwerk entspricht;

e) Anwenden der physiko-chemischen Eigenschaften bei dem neuronalen Vorhersagenetzwerk der genannten vordefinierten Gruppe, um Ausbeute- und Qualitätsparameter des unbekannten Rohöls vorherzusagen.

2. Verfahren zur Vorhersage der Rohölqualität und Rohölausbeute gemäß Anspruch 1, wobei die physiko-chemischen Eigenschaften des unbekannten Rohöls ausgewählt sind aus: API Dichte, Schwefelgehalt, Viskosität, Acidität, Kohlenstoffrest, Nickelgehalt, Vanadiumgehalt, UOP K Faktor, Asphalten, Dichte, Rauchpunkt, Gefrierpunkt, Trübungspunkt und Fließpunkt.

3. Verfahren zur Vorhersage der Rohölqualität und Rohölausbeute gemäß Anspruch 1, wobei die vorhergesagten Ausbeuten für die Fraktionen des Rohöls sich beziehen auf: Benzingehalt, Naphtagehalt, Kerosingehalt, Gasölgehalt, Vakuumdestillatgehalt und Rückstand.

4. Verfahren nach Anspruch 3, wobei für jede der Fraktionen die vorhergesagten Qualitätsparameter ausgewählt sind aus: Dichte, Schwefelgehalt, Viskosität, Acidität, Gehalt an aromatischen Verbindungen, Paraffingehalt, Naphthengehalt, Rauchpunkt, Gefrierpunkt, Trübungspunkt und Fließpunkt, Kohlenstoffrest, Asphalten, Nickelgehalt, Vanadiumgehalt.

5. Verfahren zur Vorhersage der Rohölqualität und Rohölausbeute gemäß Anspruch 1, wobei die Anzahl der in Schritt a) bestimmten Eigenschaften des Rohöls bei mindestens 4 liegt.

6. Verfahren zur Vorhersage der Rohölqualität und Rohölausbeute gemäß Anspruch 1, wobei die vordefinierte Gruppe eine Kombination von Wertebereichen der physiko-chemischen Eigenschaften des Rohöls ist.

7. Verfahren zur Vorhersage der Rohölqualität und Rohölausbeute gemäß Anspruch 1, wobei die Anzahl der vordefinierten Gruppen im Bereich von 5 bis 10 liegt.

8. Verfahren zur Vorhersage der Rohölqualität und Rohölausbeute gemäß Anspruch 1, wobei das neuronale Netzwerksystem aus neuronalen Gruppierungsnetzwerken des selbstorganisierenden Karten-Typs besteht.

9. Verfahren zur Vorhersage der Rohölqualität und Rohölausbeute gemäß Anspruch 1, wobei das neuronale Netzwerksystem aus neuronalen Vorhersagenetzwerken des Backpropagation-Typs besteht.

10. Verfahren zur Vorhersage der Rohölqualität und Rohölausbeute gemäß einem der vorhergehenden Ansprüche, wobei die Transferfunktionen der Neuronen des neuronalen Vorhersagenetzwerks des Backpropagation-Typs linear oder alternativ hyperbolische Tangenten sind.

11. Verfahren zur Vorhersage der Rohölqualität und Rohölausbeute gemäß einem der vorhergehenden Ansprüche, wobei die neuronalen Netzwerke mindestens 3 Schichten aufweisen, umfassend die Eingabe- und Ausgabeschichten.

12. Verfahren zur Vorhersage der Rohölqualität und Rohölausbeute gemäß Anspruch 1, ferner umfassend eine vorausgehende Trainingsphase.

13. Verfahren zur Vorhersage der Rohölqualität und Rohölausbeute gemäß Anspruch 12, wobei die Trainingsphase die folgenden Phasen umfasst:

i) Anwenden der physiko-chemischen Eigenschaften von bekannten Rohölen als Eingabe und der entsprechenden Ausbeuten- und Qualitätsparamter der bekannten Rohöle als Ausgabe an das neuronale Netzwerksystem;

ii) Konstruieren und Optimieren des neuronalen Gruppierungsnetzwerks mittels genetischer Algorithmen, Verteilen der Eingabedaten auf eine passende Anzahl von Gruppen, so dass jede Gruppe einem optimalen Homogenitätsgrad entspricht;

iii) für jede der genannten Gruppen konstruieren und optimieren eines neuronalen Vorhersagenetzwerks das damit verknüpft ist, unter Verwendung genetischer Algorithmen und Zuordnen der physiko-chemischen Eigenschaften und entsprechenden Ausbeuten- und Qualitätsparameter der zu dieser Gruppe gehörenden bekannten Rohöle;

iv) Validieren des neuronalen Netzwerksystems durch Minimieren der Abweichung zwischen den Werten der vorhergesagten Ausbeute- und Qualitätsparameter und der entsprechenden Werte der bekannten Rohöle.

**14.** Verfahren zur Vorhersage der Rohölqualität und Rohölausbeute gemäß Anspruch 13, wobei der optimale Homogenitätsgrad dem Kohonen-Algorithmus entspricht.

**15.** Verfahren zur Vorhersage des Rohölqualität und Rohölausbeute gemäß Anspruch 13, wobei die Eingabedaten des neuronalen Vorhersagenetzwerks die Selben wie für das neuronale Gruppierungsnetzwerk sind.

**16.** Verfahren zur Vorhersage der Rohölqualität und Rohölausbeute gemäß Anspruch 13, wobei mindestens 30 der Typen bekannter Rohöle vorliegen.

**17.** Verfahren zur Vorhersage der Rohölqualität und Rohölausbeute gemäß Anspruch 13, wobei während der Trainingsphase zufällig ausgewählte anfängliche Gewichte den neuronalen Netzwerken zugeteilt werden, welche nachfolgend angepasst und optimiert werden, bis der "r"-Korrelationskoeffizient zwischen den bekannten Werten und den vorhergesagten Werten maximiert ist.

**18.** Verfahren zur Vorhersage der Rohölqualität und Rohölausbeute gemäß Anspruch 13, wobei während der Trainingsphase zufällig ausgewählte anfängliche Gewichte den neuronalen Netzwerken zugeordnet werden, welche nachfolgend angepasst und optimiert werden, bis der Vorhersagefehler minimiert ist.

**19.** Verfahren zur Vorhersage der Rohölqualität und Rohölausbeute gemäß Anspruch 13, wobei die neuronalen Netzwerke in der Trainingsphase mit der Hilfe genetischer Algorithmen gemäß den folgenden Schritten optimiert und konstruiert werden:

1. Erzeugen einer Anfangspopulation von Genotypen;
2. Konstruieren von phänotypischen neuronalen Netzwerken basierend auf den Genotypen;
3. Trainieren und Testen der neuronalen Netzwerke, um zu bestimmen, in welchem Ausmaß sie den Unterscheidungsparametern entsprechen;
4. Vergleichen der erhaltenen Resultate, um die besten Netzwerke auszuwählen;
5. Auswählen der besten Netzwerke aus den verfügbaren Populationen und Verwerfen der schlechtesten;
6. Zurückbringen der ausgewählten Population auf einen vorab etablierten Berechnungswert gemäß einer der folgenden Subphasen:

   - Verkreuzen (intercrossing) der Genotypen der neuronalen Netzwerke;
   - Koppeln der Genotypen durch Austausch der Gene zwischen den Netzwerken;
   - Verändern der Genotypen gemäß einer Zufallsmodalität;

7. Fortsetzen des Verfahrens von Schritt 2 bis ein Stopp-Kriterium erreicht wird.

**20.** Verfahren zur Vorhersage der Rohölqualität und Rohölausbeute gemäß Anspruch 19, wobei das Stopp-Kriterium die Maximierung des "r"-Korrelationskoeffizienten zwischen den bekannten Werten und den durch das System vorhergesagten, oder alternativ die Minimierung des Fehlers ist.

**Revendications**

**1.** Procédé pour prédire la qualité et les rendements d'un pétrole brut par l'application de réseaux neuronaux et d'algorithmes génétiques, **caractérisé en ce qu'**il comprend les phases suivantes :

a) optimisation d'un système de réseaux neuronaux au moyen d'un algorithme génétique en fonction de valeurs d'entrée et de sortie connues à priori ;
b) détermination d'un nombre suffisant de caractéristiques physicochimiques d'un pétrole brut inconnu ;
c) introduction desdites caractéristiques physicochimiques dans un système de réseaux neuronaux constitué d'un réseau de groupement neuronal et d'au moins un réseau de prédiction neuronal, lesdits réseaux neuronaux étant assemblés et optimisés au moyen d'algorithmes génétiques ;

d) application desdites caractéristiques physicochimiques audit réseau de groupement neuronal pour que le pétrole brut inconnu soit associé à un groupe prédéfini auquel correspond un réseau de prédiction neuronal spécifique ;

e) application desdites caractéristiques physicochimiques audit réseau de prédiction neuronal dudit groupe prédéfini pour que soient prédits les rendements et les paramètres de qualité dudit pétrole brut inconnu.

2. Procédé pour prédire la qualité et les rendements d'un pétrole brut selon la revendication 1, dans lequel lesdites caractéristiques physicochimiques dudit pétrole brut inconnu sont choisies parmi : la densité API, la teneur en soufre, la viscosité, l'acidité, les résidus carbonés, la teneur en nickel, la teneur en vanadium, le facteur K UOP, les asphaltènes, la masse volumique, le point de fumée, le point de congélation, le point de trouble et le point d'écoulement.

3. Procédé pour prédire la qualité et les rendements d'un pétrole brut selon la revendication 1, dans lequel les rendements prédits pour les fractions de pétrole brut se réfèrent à : la teneur en gaz, la teneur en naphta, la teneur en kérosène, la teneur en gazole, la teneur en distillat sous vide et les résidus.

4. Procédé selon la revendication 3, dans lequel, pour chacune desdites fractions, les paramètres de qualité prédits sont choisis parmi : la masse volumique, la teneur en soufre, la viscosité, l'acidité, la teneur en composés aromatiques, la teneur en paraffine, la teneur en naphtène, le point de fumée, le point de congélation, le point de trouble et le point d'écoulement, les résidus carbonés, les asphaltènes, la teneur en nickel, la teneur en vanadium.

5. Procédé pour prédire la qualité et les rendements d'un pétrole brut selon la revendication 1, dans lequel le nombre de caractéristiques du pétrole brut déterminées dans l'étape a) est d'au moins 4.

6. Procédé pour prédire la qualité et les rendements d'un pétrole brut selon la revendication 1, dans lequel ledit groupe prédéfini est une combinaison de plages de valeurs desdites caractéristiques physicochimiques du pétrole brut.

7. Procédé pour prédire la qualité et les rendements d'un pétrole brut selon la revendication 1, dans lequel le nombre desdits groupes prédéfinis est situé dans la plage allant de 5 à 10.

8. Procédé pour prédire la qualité et les rendements d'un pétrole brut selon la revendication 1, dans lequel ledit système de réseaux neuronaux est constitué de réseaux de groupement neuronaux du type à carte auto-organisatrice.

9. Procédé pour prédire la qualité et les rendements d'un pétrole brut selon la revendication 1, dans lequel ledit système de réseaux neuronaux est constitué de réseaux de prédiction neuronaux du type à rétro-propagation.

10. Procédé pour prédire la qualité et les rendements d'un pétrole brut selon l'une quelconque des revendications précédentes, dans lequel les fonctions de transfert des neurones dudit réseau de prédiction neuronal du type à rétro-propagation sont linéaires ou, en variante, des tangentes hyperboliques.

11. Procédé pour prédire la qualité et les rendements d'un pétrole brut selon l'une quelconque des revendications précédentes, dans lequel lesdits réseaux neuronaux comportent au moins 3 couches, y compris les couches d'entrée et de sortie.

12. Procédé pour prédire la qualité et les rendements d'un pétrole brut selon la revendication 1, comprenant également une phase d'apprentissage préliminaire.

13. Procédé pour prédire la qualité et les rendements d'un pétrole brut selon la revendication 12, dans lequel ladite phase d'apprentissage comprend les phases suivantes :

i) application audit système de réseaux neuronaux des caractéristiques physicochimiques de pétroles bruts connus en entrée et les rendements et paramètres de qualité correspondants de pétroles bruts connus en sortie ;

ii) assemblage et optimisation dudit réseau de groupement neuronal au moyen d'algorithmes génétiques, distribution des données d'entrée dans un nombre commode de groupes, de façon que chaque groupe corresponde à un niveau d'homogénéité optimal ;

iii) pour chacun desdits groupes, assemblage et optimisation d'un réseau de prédiction neuronal associé à celui-ci, utilisation d'algorithmes génétiques et attribution des caractéristiques physicochimiques et des rendements et paramètres de qualité correspondants desdits pétroles bruts connus en relation avec ledit groupe ;
iv) validation dudit système de réseaux neuronaux par minimisation de l'écart entre les valeurs des rendements et paramètres de qualité prédits et les valeurs correspondantes des pétroles bruts connus.

**14.** Procédé pour prédire la qualité et les rendements d'un pétrole brut selon la revendication 13, dans lequel ledit niveau d'homogénéité optimal correspond à l'algorithme de Kohonen.

**15.** Procédé pour prédire la qualité et les rendements d'un pétrole brut selon la revendication 13, dans lequel les données d'entrée du réseau de prédiction neuronal sont les mêmes que celles du réseau de groupement neuronal.

**16.** Procédé pour prédire la qualité et les rendements d'un pétrole brut selon la revendication 13, dans lequel il y a au moins 30 desdits types de pétroles bruts connus.

**17.** Procédé pour prédire la qualité et les rendements d'un pétrole brut selon la revendication 13, dans lequel, durant ladite phase d'apprentissage, des poids initiaux sélectionnés au hasard sont attribués auxdits réseaux neuronaux, qui sont ensuite ajustés et optimisés jusqu'à ce que le coefficient de relation "r" entre les valeurs connues et les valeurs prédites soit maximisé.

**18.** Procédé pour prédire la qualité et les rendements d'un pétrole brut selon la revendication 13, dans lequel, durant ladite phase d'apprentissage, des poids initiaux sélectionnés au hasard sont attribués auxdits réseaux neuronaux, qui sont ensuite ajustés et optimisés jusqu'à ce que l'erreur de prédiction soit minimisée.

**19.** Procédé pour prédire la qualité et les rendements d'un pétrole brut selon la revendication 13, dans lequel lesdits réseaux neuronaux dans la phase d'apprentissage sont optimisés et assemblés à l'aide d'algorithmes génétiques conformément aux étapes suivantes :

1. création d'une population initiale de génotypes ;
2. assemblage de réseaux neuronaux phénotypiques sur la base des génotypes ;
3. apprentissage et test des réseaux neuronaux pour déterminer dans quelle mesure ils correspondent au paramètre discriminant ;
4. comparaison des résultats obtenus pour sélectionner les meilleurs réseaux ;
5. sélection des meilleurs réseaux parmi les populations disponibles et mise à l'écart des pires ;
6. renvoi de la population sélectionnée à une valeur de calcul préétablie en fonction de l'une des sous-phases suivantes :

   - entrecroisement des génotypes des réseaux neuronaux ;
   - couplage des génotypes par l'échange de gènes entre les réseaux ;
   - changement des génotypes en fonction d'une modalité aléatoire ;

7. reprise du procédé à partir de l'étape 2 jusqu'à ce qu'un critère d'arrêt soit atteint.

**20.** Procédé pour prédire la qualité et les rendements d'un pétrole brut selon la revendication 19, dans lequel ledit critère d'arrêt est la maximisation du coefficient de corrélation "r" entre les valeurs connues et celles prédites par le système ou en variante la minimisation de l'erreur.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5699269 A
- US 6477516 B
- US 5572030 A

**Non-patent literature cited in the description**

- **BLANCO M et al.** Geographical origin classification of petroleum crudes from near-infrared spectra bitumens. *Applied Spectroscopy,* 2001, 834-839
- **BLANCO M. et al.** Determination of physical properties of bitumen by use of near-infrared spectroscopy with neural networks. Joint modeling of linear and non-linear parameters. *Analyst,* 2001, vol. 126, 378-382